(19) Europäisches Patentamt
European Patent Office
Office européen des brevets

(11) Veröffentlichungsnummer: **0 277 473 B1**

(12) **EUROPÄISCHE PATENTSCHRIFT**

(45) Veröffentlichungstag der Patentschrift: **12.04.95**

(51) Int. Cl.$^{6}$: **C08F 8/00**, C12N 11/08

(21) Anmeldenummer: **88100006.1**

(22) Anmeldetag: **04.01.88**

# (54) Reversibel ausfällbare, wasserlösliche Polymerkonjugate, ihre Herstellung und Verwendung in der homogenen Katalyse.

(30) Priorität: **08.01.87 DE 3700308**

(43) Veröffentlichungstag der Anmeldung:
**10.08.88 Patentblatt 88/32**

(45) Bekanntmachung des Hinweises auf die Patenterteilung:
**12.04.95 Patentblatt 95/15**

(84) Benannte Vertragsstaaten:
**AT BE CH DE FR GB IT LI NL SE**

(56) Entgegenhaltungen:
**EP-A- 0 058 767**
**DE-A- 2 237 316**
**DE-A- 2 525 727**
**DE-A- 2 546 242**
**US-A- 4 593 073**

**Patent Abstracts of Japan, Band 10, nr. 17, 23 Januar 1986; & JP-A-60170609**

(73) Patentinhaber: **HOECHST AKTIENGESELL-SCHAFT**

**D-65926 Frankfurt (DE)**

(72) Erfinder: **Vorlop, Klaus-Dieter, Dr.**
**Hochstrasse 7**
**D-3300 Braunschweig (DE)**
Erfinder: **Steinke, Kerstin**
**Frankfurter Strasse 268**
**D-3300 Braunschweig (DE)**
Erfinder: **Wullbrandt, Dieter, Dr.**
**Bienerstrasse 29**
**D-6238 Hofheim am Taunus (DE)**
Erfinder: **Schlingmann, Merten, Prof. Dr.**
**Schneidhainer Strasse 32a**
**D-6240 Königstein/Taunus (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft aus wäßriger Lösung reversibel ausfällbare Polymerkonjugate, bestehend aus einem im wäßrigen Medium löslichen Trägerpolymeren und einer daran chemisch gebundenen, katalytisch wirksamen Verbindung. Gleichermaßen betrifft die Erfindung ein Verfahren zur Herstellung solcher Polymerkonjugate und die Verwendung derselben in der homogenen Katalyse, insbesondere der Biokatalyse.

Trägergebundene Katalysatoren, insbesondere trägergebundene (immobilisierte) Enzyme haben eine Vielzahl von Anwendungen, z.B. in der medizinischen Analytik, bei der Herstellung von Produkten aus Pharmazie (Antibiotika) und Pflanzenschutz, bei der Herstellung von Nahrungsmitteln sowie bei der Gewinnung optisch aktiver Substanzen (Racematspaltung bei Aminosäuren).

Ein wirtschaftlicher Einsatz solcher Katalysatoren ist jedoch vorteilhaft, wenn der meist recht teure Katalysator zurückgewonnen, d.h. mehrfach eingesetzt werden kann. Als Lösung bietet sich die Fixierung (Immobilisierung) solcher katalytisch wirksamen Verbindungen an. In dieser Form sind sie unter Wahrung ihrer katalytischen Eigenschaften leicht handhabbar. Folgende Immobilisierungsmethoden sind beschrieben:

1. Immobilisierung von katalytisch wirksamen Verbindungen an oder in Feststoffpartikeln (heterogene Katalyse) durch:
   - Einschluß in eine (polymere) Matrix
   - Adsorption
   - Kovalente Bindung
   - Mikroverkapselung
2. Immobilisierung von katalytisch wirksamen Verbindungen durch kovalente Bindung an wasserlösliche Polymere (homogene Katalyse).

Die fixierten Katalysatoren sind häufig gegenüber der freien Form stabilisiert.

Der Einsatz wasserlöslicher Trägerpolymere wie Polyethylenglykole zur Immobilisierung von katalytisch aktiven Verbindungen findet Anwendung zur Stabilisierung und Molekulargewichtsvergrößerung von Enzymen und Cofaktoren in Membranreaktoren.

Bei Reaktionen von hochmolekularen wasserunlöslichen Substraten bzw. in Reaktionen mit unlöslichen Produkten, ist der Einsatz heterogener Katalysatoren bzw. Membranreaktoren nicht geeignet. Die Entwicklung eines wasserlöslichen, immobilisierungsfähigen Polymers, das durch Ausfällung zurückzugewinnen ist, würde sowohl die Verarbeitung hochmolekularer Substanzen als auch Reaktionen mit wasserunlöslichen Produkten möglich machen.

Es ist eine Vielzahl von Methoden zur Immobilisierung von katalytisch wirksamen Verbindungen, insbesondere Enzymen, bekannt. Eine ausführliche Übersicht wird beispielsweise in Methods in Enzymology, Vol. XLIV, "Immobilized Enzymes", (Academic Press, 1976) und in J. Chibata: Immobilized Enzymes (Kodansha Ltd., John Wiley & Sons, 1978) gegeben. Ein häufig beschriebener Weg ist die adsorptive, ionische oder kovalente Bindung von Enzymen an Träger.

In der DE-AS 2 237 316 werden als Trägersubstanzen quellbare, vernetzte Perlpolymerisate beschrieben, die durch Copolymerisation von reaktiven Gruppen enthaltenden Monomeren, vernetzenden Monomeren und hydrophilen Monomeren erhalten werden. Als reaktive Gruppe werden dabei die Halogenalkyl-, die Epoxid-, die Carbonsäurechlorid-, Carbonsäureanhydrid-, Carbonsäureazid-, Carbonsäurephenylester- und Hydroxamsäure-Gruppe offenbart.

In der DE-OS 27 22 751 wird die Herstellung von Perlpolymerisaten als Trägerpolymer aus Acryl-/Methacrylamid und/oder Methylen-bis-acryl-/methacrylamid beschrieben. Gegebenenfalls enthalten diese Polymeren noch weitere, radikalisch polymerisierbare Comonomere mit einer Oxiran-, Carbonsäureanhydrid-, Carbonsäurechlorid-, Carbonsäureazid-, Carbonsäurephenylester- oder Hydroxamsäure-Gruppe.

In der DE-OS 2546 242 werden Polymere beschrieben, die dadurch gekennzeichnet sind, daß Reste der Nicotinsäure vorhanden sind, die mit einer Polymeren Matrix so verbunden sind, daß sie in biologischer Umgebung hydrolysiert werden und freie Nicotinsäure bilden können.

Alle diese literaturbekannten Trägerpolymeren haben jedoch den Nachteil, daß sie nicht wasserlöslich sind. Zwar lassen sich Enzyme oder andere katalytisch aktive Substanzen chemisch an diese Polymere binden, jedoch wird deren Aktivität bei weitem nicht ausgenutzt, da die aktive Oberfläche im Vergleich zu einem gelösten Polymer mit chemisch gebundenen katalytisch wirksamen Verbindungen noch relativ gering ist.

Es wurden nun wasserlösliche Polymerkonjugate gefunden, die aufgrund von Temperaturerhöhung und/oder Salzzugabe ausflocken und somit von einer Reaktionslösung durch einfache physikalische Scheidemethoden abgetrennt werden können. Dabei läßt sich der Fällungspunkt dieser Polymerkonjugate in einem weiten Temperaturbereich (ca. 10 bis 80 °C) einstellen und auf die jeweilige Arbeitstemperatur des chemisch gebundenen, katalytisch aktiven Bausteins abstimmen. Die Einstellung des Fällungspunktes

erfolgt durch Variation der Monomerzusammensetzung. Die Flockungstemperatur wählt man vorteilhaft so, daß sie nur wenig oberhalb der günstigsten Arbeitstemperatur des gebundenen katalytisch wirksamen Bausteine liegt.

Die Erfindung betrifft somit:

Wasserlösliches Polymerkonjugat, bestehend aus einem Trägerpolymeren und einer chemisch daran gebundenen katalytisch aktiven Verbindung, das dadurch gekennzeichnet ist, daß das Polymerkonjugat aus einem wasserlöslichen, aus wäßriger Lösung durch Temperaturerhöhung - bevorzugt im Bereich von 10 - 80 °C - und/oder Salzzugabe reversibel ausfällbaren Trägerpolymeren, welches aus mindestens einer wiederkehrenden Monomereinheiten der Formel I

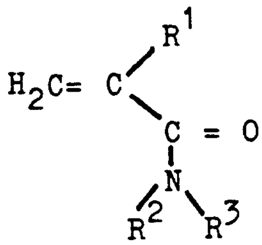 (I)

in denen $R^1$ und $R^3$ gleich oder verschieden sind und Wasserstoff oder geradkettiges oder verzweigtes $C_1$-$C_3$-Alkyl und $R^2$ geradkettiges oder verzweigtes $C_1$-$C_3$-Alkyl bedeutet, und mindestens einer weiteren wiederkehrenden Monomereinheit ausgewählt aus den Verbindungen der Formel II, III und IV

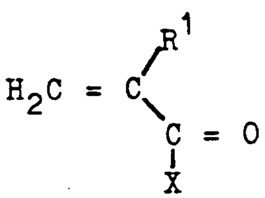 (II) 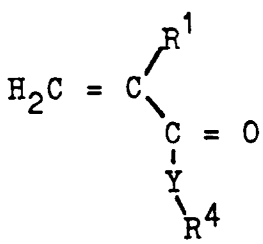 (III) 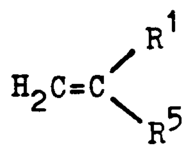 (IV)

wobei

$R^1$ wie zu Formel I angegeben definiert ist

$R^4$ eine hydrophobe Gruppe ist und geradkettiges $C_1$-$C_6$-Alkyl, welches im Falle der $C_3$-$C_6$-Alkylreste auch verzweigt oder cyclisch sein kann

$R^5$ eine aktivierte Gruppe ist und 3,4-Epoxicyclohexyl oder ein Substituent der Formel V,

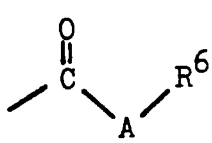 (V)

wobei

A Sauerstoff, Schwefel oder eine NH-Gruppe und

$R^6$ ein $-(CH_2)_n$-Z-Rest,

worin

n eine Zahl von 1 bis 6 und

Z Amin, Hydroxyd, Carboxyl, Carbonyl oder 1,2-Epoxyethan

bedeutet,

ist,

X eine hydrophile Gruppe ist und eine OH-, $NH_2$- oder SH-Gruppe und

Y Sauerstoff oder Schwefel

bedeutet, besteht und aus einer Katalytisch aktiven Verbindung besteht.

Unter katalytisch aktiven Verbindungen werden in den vorstehenden und folgenden Ausführungen Enzyme, Coenzyme, Antigene, Antikörper, Metalle oder Metallkomplexe (Chiralika), Aminosäuren und Affinitätsmaterialien verstanden; insbesondere Enzyme.

Weiterhin betrifft die Erfindung ein Verfahren zur Herstellung der erfindungsgemäßen Polymerkonjugate, das dadurch gekennzeichnet ist, daß ein wasserlösliches, aus wäßriger Lösung durch Temperaturerhöhung und/oder Salzzugabe reversibel ausfällbares Trägerpolymer mit einer katalytisch aktiven Verbindung umgesetzt wird, gegebenenfalls nach vorangegangener chemischer Modifizierung der an dem Trägerpolymer vorhandenen Seitenketten.

Schließlich betrifft die Erfindung die Verwendung der erfindungsgemäßen Polymerkonjugate in der homogenen Katalyse, insbesondere in der homogenen Biokatalyse.

Im folgenden wird die Erfindung näher beschrieben.

Das Trägerpolymere muß folgende Voraussetzungen erfüllen:

a) Vollständig in 1-20 °C warmem Wasser löslich

b) ausfällbar durch Temperaturerhöhung, bevorzugt im Temperaturbereich von 10-80 °C und/oder Salzzugabe

c) vollständig wiederauflösbar wenn die Temperatur bzw. die Salzkonzentration gesenkt wird (reversibel ausfällbar)

d) auch in Gegenwart anderer löslicher Polymere oder Feststoffen vollständig und einfach abtrennbar.

Von den Trägerpolymeren sind solche bevorzugt, die aus wiederkehrenden Monomereinheiten gemäß Formel I und mindestens einer weiteren wiederkehrenden Monomereinheit, ausgewählt aus den Verbindungen der Formel II, III und IV besteht.

Besonders bevorzugt sind die Polymere, in denen

$R^1$ Wasserstoff, Methyl oder Ethyl,
$R^2$ Ethyl, n- oder i-Propyl,
$R^3$ Wasserstoff, Methyl oder Ethyl
$R^4$ geradkettiges oder verzweigtes $C_1$-$C_4$-Alkyl,
$R^5$ 3,4-Epoxycyclohexan oder ein Substituent der Formel V,
wobei
$R^6$ ein -$(CH_2)_n$-Z-Rest mit n = 1 bis 3 und
Z Carbonyl oder 1,2-Epoxyethan bedeutet und
A Sauerstoff oder eine NH-Gruppe ist,
X eine OH- oder $NH_2$-Gruppe und
Y Sauerstoff

bedeuten.

Ganz besonders bevorzugt besteht das Trägerpolymer aus: N-Isopropylacrylamid oder N-Isopropylmethacrylamid (gemäß Formel I)
und einer Verbindung, ausgewählt aus Methacrylamid, Acrylsäuremethylester
und Glycidylmethacrylat oder Glycidylacrylat (gemäß den Formeln II, III und IV).

Zweckmäßigerweise enthält das Trägerpolymer nur eine der hydrophilen bzw. hydrophoben Comonomer-Komponenten. Es ist jedoch auch durchaus möglich, Gemische dieser Komponenten einzusetzen.

Die bevorzugt eingesetzten Trägerpolymere enthalten 0,2 - 80 Gew.-%, bezogen auf den Gewichtsanteil des Polymers gemäß Formel I, der Comonomerbausteine ausgewählt aus den Verbindungen der Formel II, III und IV, wobei auch Kombinationen aller drei Substanzen zugelassen sind. Bevorzugt enthalten sie 0,2 - 40 Gew.-%, besonders bevorzugt 5 - 40 Gew.-% dieser Comonomeren. Werden Kombinationen aus den Verbindungen gemäß Formel II, III und IV eingesetzt, so beziehen sich die angegebenen Werte auf deren Gesamtgewicht.

Sofern Comonomerbausteine gemäß Formel IV alleine oder im Gemisch mit anderen in dem Trägerpolymer vorhanden sind, beträgt ihr Gewichtsanteil an dem Gewicht des Restpolymers bevorzugt 0,2-15 %. Besonders bevorzugt ist ein Gewichtsanteil von 0,2-3 %.

Zwischen der Ausfälltemperatur und dem Gehalt an hydrophilem bzw. hydrophobem Comonomer besteht ein linearer Zusammenhang. Aus Figur 1, die den prozentualen Gehalt (bezogen auf das fertige Polymer) an Copolymer zeigt, geht diese Relation, die hier beispielhaft für Poly-N-i-propylacrylamid PIPAM mit variierenden Anteilen an Acrylsäuremethylester AME und Methacrylamid MAA dargestellt ist, eindeutig hervor.

Auf diese Weise ist es möglich, Trägerpolymere je nach Anwendung mit einer definierten Fällungstemperatur herzustellen.

Die für die Polymerisation benötigten Monomere können, sofern sie nicht käuflich sind, auf einfache Weise hergestellt werden. Beispielsweise erhält man die N-alkylsubstituierten Acryl-/Methacrylamide (gemäß Formel I oder II) durch Umsetzung von Acryl-/Methacrylsäurehalogenid, bevorzugt Acryl-/Methacrylsäurechlorid, mit den gewünschten Alkyl- oder Dialkylaminen.

Die monomeren Acrylate gemäß Formel III und IV werden z.B. durch Umsetzung von Acryl-/Methacrylsäurehalogeniden, bevorzugt -chloriden, mit den entsprechenden Alkoholen oder Mercaptanen hergestellt.

Die Monomeren gemäß Formel V können beispielsweise durch Umsetzung von Acryl-/Methacrylsäurehalogeniden, bevorzugt -chloriden mit Alkoholen, Mercaptanen oder primären Aminen, wobei der Substituent Z gegebenenfalls vor dieser Umsetzung mit einer geeigneten Schutzgruppe z.B. Ester, Acetal, Ether oder Tosylat geschützt wird, hergestellt werden.

Die entsprechenden höheren Alkylacrylamide erhält man beispielsweise aus den 2-Ethyl- bzw. 2-Propyl-/i-Propylacroleinen durch Oxidation z.B. mit Chromsäure zu den entsprechenden Acrylsäuren (s. Organikum, Organisch Chemisches Grundpraktikum 15. überarbeitete Auflage, S. 447, VEB Deutscher Verlag der Wissenschaften, Berlin 1976), die anschließend zu den Carbonsäurehalogeniden umgesetzt werden, welche dann wie oben beschrieben mit Alkoholen oder Aminen zu den gewünschten Alkylacrylamiden/-säuren reagieren.

Die Trägerpolymere werden durch radikalische Polymerisation der Monomereinheiten in einem Lösungsmittel, vorzugsweise Wasser, erhalten.

Die Polymerisation wird in an sich bekannter Weise, z.B. durch UV-Licht, energiereiche Strahlung, in der Regel aber durch einen im Monomergemisch löslichen, Radikale liefernden Initiator ausgelöst. Geeignete Initiatoren sind z.B. lösliche Peroxodisulfate, Benzoylperoxid, tert.-Butylhydroperoxyd, Cumolperoxyd, Methyläthylketonperoxyd, Lauroylperoxyd, tert.-Butylperbenzoat, tert.-Butyldiperphthalat, Azodiisobutyronitril, 2,2'-Azobis-(2,4-dimethylvaleronitril), 2-Phenyl-azo-2,4-dimethyl-4-methoxy-valeronitril, 2-Cyano-2-propyl-azoformamid, Azodiisobutylamid, Dimethyl-, Diäthyl-, oder Dibutyl-azobis-methylvalerat. Bevorzugt wird ein Ammonium-peroxodisulfat/Natriumthiosulfit-Gemisch eingesetzt. Bezogen auf die Monomermenge werden etwa 0,01 bis 2 Gew.-%, vorzugsweise 0,1 bis 1 Gew.-% Initiator angewandt.

Die Reaktion wird zweckmäßig unter Sauerstoffausschluß in einer Inertgasatmosphäre, wie Stickstoff oder Argon, durchgeführt. Die Reaktionszeiten betragen 10 Minuten bis 10 Stunden, bevorzugt 30 Minuten bis 5 Stunden. Die Polymerisation kann beispielsweise durch Einblasen von Luft oder Sauerstoff zu jedem beliebigen Zeitpunkt abgebrochen werden. Bevorzugt wird die Reaktion dann abgebrochen, wenn keine Viskositätszunahme mehr festzustellen ist. Die Reaktionstemperatur liegt zwischen 0 und 40 °C, bevorzugt zwischen 0 und 20 °C.

Zur Herstellung der bevorzugt eingesetzten Trägerpolymeren werden - bezogen auf die vorgelegte Menge an Monomersubstanz gemäß Formel I-0,2-80 Gew.-%, bevorzugt 0,2 - 60 Gew.-% mindestens eines Monomers, ausgewählt aus den Verbindungen der Formel II, III und IV, hinzugegeben. Die hinzugegebene Menge an Comonomer kann auch aus beliebigen Gemischen der Monomere gemäß Formel II, III und IV bestehen; für eine möglichst exakte Einstellung der Fällungstemperatur ist es jedoch vorteilhafter, nur eine Comonomerkomponente gemäß Formel II oder III einzusetzen.

Soll das Trägerpolymer Monomereinheiten gemäß Formel IV enthalten, so wird dieses Monomer zu 0,2-20 Gew.-%, bevorzugt 0,2 - 10 Gew.-%, bezogen auf die Menge der restlichen, vorgelegten Monomeren, hinzugegeben.

Die Gesamtkonzentration aller Monomeren in dem Lösungsmittel beträgt 1 - 30 Gew.-%, bevorzugt 5 - 15 Gew.-%.

Die endgültige prozentuale Zusammensetzung des Polymerisats läßt sich erst durch quantitative Elementaranalyse bestimmen. Eine exakte Korrelation zwischen eingesetzten Monomeren und Gehalt dieser Monomer-Einheiten im Polymerisat ist nur schwer herzustellen, da die Endzusammensetzung von vielen Parametern, wie Reaktionstemperatur, -zeit und -ausbeute abhängt. Aus diesem Grund sind in der Figur 1 die prozentualen Anteile der Monomer-Einheiten im Trägerpolymeren und nicht die Zusammensetzung der Monomeren vor der Reaktion, aufgetragen.

Die oben beschriebenen Trägerpolymere werden nun mit katalytisch wirksamen Substanzen zu den erfindungsgemäßen Polymerkonjugaten in an sich bekannter Weise umgesetzt. Unter katalytisch wirksamen Verbindungen werden solche Substanzen verstanden, die in der Lage sind, einen mit ihnen in Kontakt gebrachten Reaktionspartner chemisch zu verändern, ohne selbst dabei eine permanente, strukturelle oder chemische Veränderung zu erfahren. Sie gehen aus der Reaktion also selbst unverändert hervor. Als Beispiele für solche katalytisch aktiven Bausteine seien genannt:

a) Metalle oder Metallkomplexe (Chiralika) z.B. für enantioselektive Hydrierung, Polymerisation oder Hydroformylierung, Hydrosilylierung (homogene Übergangs- und Edelmetall-Katalyse) beispielsweise seien hier genannt:

Phosphankomplexe von $[RhCl(PPh_3)_3]$ vgl. DE-PS 92 031 $[Co_2(CO)_8]$ vgl. US 3 998 864 $[Na_2PdCl_4]$ vgl. DE-PS 2 330 308 $[Rh_2Cl_2(C_2H_4)_4]$ vgl. DD-PS 133 199 Ph = Phenyl

für die Herstellung reiner optischer Antipoden, insbesondere Aminosäuren beispielsweise seien hier genannt:
Phosphatkomplexe von: $[Rh_2Cl_2(C_2H_4)_4]$ DE-PS 27 18 533 $[Rh_2Cl_2(Cod)_2]$ DE-PS 28 24 861 $[Rh(Norbarn)_2]ClO_4$ JP-PS 59 93 090
Cod = Cyclooctadien, Norban = Norbornadien
Bei den genannten Metallkomplexen sind die Übergangsmetalle meist ihrerseits an organische Reste mit endständigen $-PPh_2$-Gruppen koordinativ gebunden. Diese organischen Reste können auf die gleiche Weise an die aktive Seitenkette der oben beschriebenen Trägerpolymere gebunden werden, wie dies in einer Vielzahl der oben angegebenen Literaturstellen für Harze oder sonstige wasserunlösliche Trägerpolymere beschrieben ist.
b) Affinitätsmaterialien
z.B. zur Isolierung von gegebenenfalls hochmolekularen Enzymen
unter Affinitätsmaterialien werden Substrate oder Substratanaloga verstanden, die kovalent an einen Polymerträger gebunden werden können und mit ihrer "freien" Seite an ein artspezifisches Enzym ankoppeln, das nach Abtrennung des Trägerpolymer-Substrat-Enzym-Konjugats vom Reaktionsmedium wieder leicht vom Substrat abgespalten und auf diese Weise isoliert werden kann. Dies wird z.B. beschrieben in:
US-PS 3 941 657 (D-Aminosäureoxidase)
Immobilized Enzymes, Preparation and Engineering, Recent Advances 1979, J.C. Johnson Solid Phase Biochemistry, Analytic and Synthetic Aspects, W.H. Scouten, Volume 66
c) Aminosäuren
z.B. für die Proteinsynthese (analog dem Merrifield-Harz) siehe z.B. Solid Phase Biochemistry, Analytic & Synthetic Aspects, W.H. Scouten, Vol. 66
d) Antikörper
e) Antigene
f) Enzyme
z.B. aus der Gruppe der Hydrolasen wie:

| | |
|---|---|
| Proteasen: | α-Chymotrypsin, Trypsin, Thermolysin, Pepsin |
| Acylasen: | Lipasen z.B. aus Candida cylindracea oder aus Schweinepankreas |
| Esterasen: | Schweineleberesterase,<br>Cholinesterasen |
| Amidasen: | Penicillinacylase |

g) Coenzyme
z.B. Adenosintriphosphat (ATP) oder Nicotinamid-Adenin-Dinucleotid (NAD-NADH)
US-PS 3 957 748

Besonders geeignet sind Enzyme und Coenzyme. Von den Enzymen lassen sich besonders gut die Proteasen und Acylasen immobilisieren. Eine Möglichkeit, katalytisch aktive Verbindungen dauerhaft an einen Träger zu koppeln, ist die kovalente Bindung. Dazu müssen die reaktiven Gruppen des Trägerpolymers und des katalytisch aktiven Bausteins, beispielsweise über eine Amin- oder Hydroxyfunktion miteinander reagieren. Dies geschieht a) durch direkte Umsetzung des gelösten Trägerpolymers mit der katalytisch aktiven Verbindung oder b) für den Fall, daß die Reaktionsbedingungen der Umsetzung für die katalytisch aktive Verbindung nicht geeignet sind - nach vorheriger chemischer Modifizierung der reaktiven Seitenkette des Trägerpolymers oder c) unter gleichzeitiger Zugabe eines "Reaktionsvermittlers".

Ein Beispiel für die direkte Umsetzung des Trägerpolymers mit einem katalytisch wirksamen Baustein ist im folgenden Reaktionsschema a) dargestellt

a)

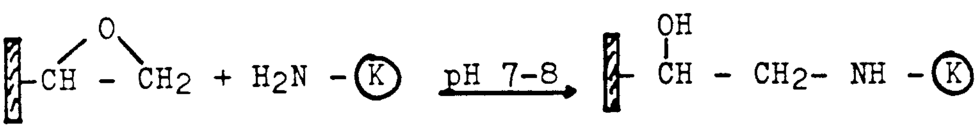


Als epoxydhaltige Trägerpolymere können beispielsweise die Polymere eingesetzt werden, die eine oxiranhaltige Seitenkette aufweisen, z.B. 1,2-Epoxyethane oder 3,4-Epoxycyclohexane.

Für solche katalytisch aktiven Verbindungen, die sich unter den Reaktionsbedingungen von a) nicht ankoppeln lassen ist es vorteilhaft, die reaktive Seitenkette des Trägerpolymers vor der Umsetzung so zu verändern (chemisch zu modifizieren), daß die Reaktion unter milderen Bedingungen stattfinden kann

(Reaktionsschema b)). Beispielsweise kann die Epoxydgruppe zunächst hydrolytisch zu einem Diol gespalten werden und anschließend z.B. mit einem Halogencyan, insbesondere Bromcyan zu einem Imidokohlensäurediester umgesetzt werden, der dann unter milden Bedingungen mit der entsprechenden katalytisch aktiven Verbindung umgesetzt werden kann (s. Reaktionsschema b).

b)

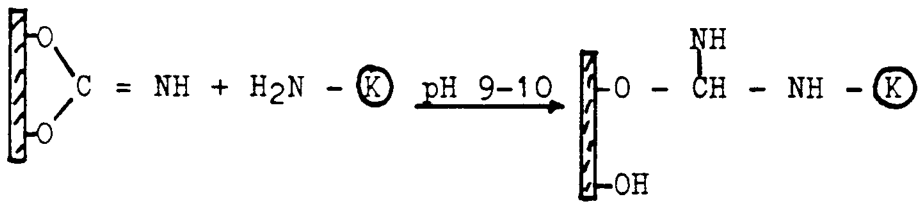


Auch in diesem Fall kann wieder von epoxydhaltigen Trägerpolymeren ausgegangen werden.

Polymere mit carbonsäurehaltiger Seitenkette können beispielsweise in Anwesenheit von Dialkylcarbodiimiden, insbesondere Dicyclohexylcarbodiimid (DCC), mit dem katalytisch aktiven Baustein umgesetzt werden (Reaktionsschema c). DCC dient in diesem Falle als "Reaktionsvermittler", erleichtert also die Umsetzung der Carbonsäure mit der Amino- oder Hydroxyfunktion des katalytisch aktiven Bausteins. Die Reaktion verläuft nach folgendem Schema:

c)

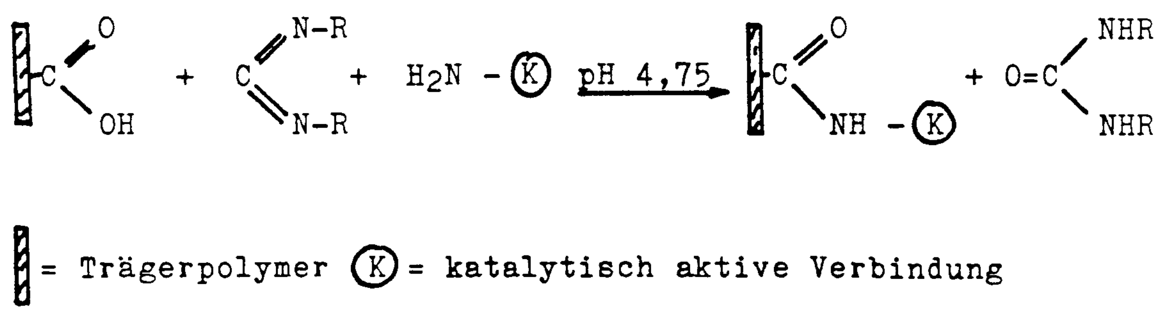


R = $C_1$-$C_8$-Alkyl/-Cycloalkyl

Trägerpolymere mit Amino-, Hydroxy- oder Carbonylgruppen in den reaktiven Seitenketten lassen sich entweder direkt nach Reaktionsschema a) oder nach einer der Varianten b) oder c) mit dem katalytisch aktiven Baustein beladen.

Zur Ankopplung der katalytisch aktiven Verbindungen an das funktionalisierte Trägerpolymere geht man am besten so vor, daß man zunächst das Trägerpolymere in einem wäßrigen Lösungsmittel, bevorzugt Wasser, auflöst und dann die entsprechende katalytisch aktive Verbindung, entweder als Reinsubstanz oder ebenfalls in wäßrigem Milieu, bevorzugt in Wasser gelöst, hinzugibt. Der pH-Wert der Lösung sollte dabei bevorzugt in dem Bereich gehalten werden, in dem die entsprechende katalytisch aktive Substanz und das Trägerpolymere stabil ist. Dies geschieht zweckmäßig durch geeignete Puffer. Vorzugsbereiche für die verschiedenen Verfahrensvarianten a), b) und c) sind in den jeweiligen Schemata angegeben. Die Reaktionszeit liegt zwischen 0,1 und 70 Stunden, bevorzugt 10 bis 60 Stunden, insbesondere 20 bis 55 Stunden. Die Reaktionstemperatur sollte zwischen 1 und 35 °C, bevorzugt 3 und 30 °C, insbesondere 5 und 25 °C liegen.

Die molaren Verhältnisse von eingesetztem, gegebenenfalls funktionalisiertem Trägerpolymer und katalytisch aktiver Verbindung sollten zweckmäßig so bemessen sein, daß pro Mol aktivierter Seitenkette mindestens ein Mol katalytisch aktive Verbindung vorhanden ist, so daß bei vollständigem Umsatz alle verfügbaren reaktiven Seitenketten mit der katalytisch aktiven Verbindung substituiert sind. Um zu einem möglichst vollständigen Umsatz zu gelangen, ist es jedoch vorteilhaft, einen Überschuß an katalytisch aktiver Substanz einzusetzen. Es kann jedoch, je nach Verwendungszweck, auch durchaus zweckmäßig sein, nicht sämtliche vorhandenen reaktiven Seitenketten mit den katalytisch aktiven Verbindungen umzusetzen, d.h. man setzt die katalytisch aktiven Verbindungen im Unterschuß ein.

Das erfindungsgemäße Polymerkonjugat wird schließlich ausgefällt, von der Lösung abgetrennt und gegebenenfalls durch ein- oder mehrfaches Umfällen gereinigt. Das Ausfällen geschieht durch Temperaturerhöhung und/oder Salzzugabe.

Das Lösungsverhalten der erfindungsgemäßen Polymerkonjugate in Wasser wird von zwei Strukturelementen, der hydrophilen und der hydrophoben Seitenkette des Trägerpolymers bestimmt.

Die thermische Ausfällung der erfindungsgemäßen Polymerkonjugate ist vollständig reversibel. Einmal erreicht, ist sowohl die gelöste als auch die ausgefällte Form des Polymers relativ stabil.

Es bestehen nun zwei Möglichkeiten zur Ausfällung des Polymerkonjugats. Entweder erhöht man die Temperatur bei gegebener Salzkonzentration, oder man setzt bei gegebener Temperatur (z.B. Raumtemperatur) der Lösung Salz zu. In beiden Fällen kommt es zur Phasentrennung, wenn durch den Einfluß der Temperatur oder der Salze die Solvathülle so stark destabilisiert ist, daß der Einfluß der hydrophoben Gruppen dominiert.

In Gegenwart von Salz, z.B. NaCl verringert sich die Wasserlöslichkeit des Polymerkonjugats. Es zeigt sich, daß ein linearer Zusammenhang zwischen der Salzkonzentration und der Löslichkeit des Polymerkonjugats besteht.

Für jede Temperatur, die kleiner als die kritische Lösungstemperatur des Polymerkonjugats in reinem Wasser ist, kann durch Zugabe einer kritischen Salzkonzentration die Solvathülle der Makromoleküle so weit abgebaut, d.h. destabilisiert werden, daß es zur Phasentrennung kommt.

Jedes Salz, bzw. jedes Ion besitzt ein charakteristisches Flockungsvermögen. Stark solvatisierte Ionen haben eine große dehydratisierende Wirkung und bewirken schon in kleiner Konzentration eine Ablösung der makromolekularen Solvathülle. Um die gleiche Wirkung mit schwach solvatisierten Ionen zu erzielen, müssen diese in größerer Menge zugesetzt werden. Fällungsversuche mit $Na^+$- und $NH_4{}^+$ -Ionen bestätigen dies. Das Flockungsvermögen weiterer Ionen kann aus der lyotropen Reihe von F. Hofmeister (R. Brdička, Grundlagen der physikalischen Chemie, 15. Auflage, VEB Deutscher Verlag der Wissenschaften, Berlin 1982, S. 668-672) abgeschätzt werden:

Reihenfolge mit abnehmendem Fällungsvermögen

Anionen: $CO_3^{2-}$ , $SO_4^{2-}$ , $PO_4^{3-}$ , Acetat$^-$, $Cl^-$, $Br^-$, $J^-$

Kationen: $Mg^{2+}$, $Ca^{2+}$, $Na^+$, $K^+$, $NH_4^+$

Erfindungsgemäß eignen sich sämtliche mögliche Kombinationen von Kat- und Anionen aus obiger Tabelle, z.B. Calciumchlorid, Natriumsulfat, Natriumacetat, Natriumchlorid, Natriumbromid, Natriumjodid, Kaliumchlorid, Kaliumbromid, Kaliumjodid, Kaliumsulfat, insbesondere Natriumchlorid.

Auch in Gegenwart von Salzen ist die Ausflockung des erfindungsgemäßen Polymerkonjugats vollständig reversibel. Ändert man die Parameter Temperatur und/oder Salzkonzentration so daß der hydrophile Einfluß überwiegt, wird das Polymerkonjugat wieder vollständig gelöst.

In Figur 2, die die Abhängigkeit der Trübung von der Temperatur zeigt, ist dies anhand einer 0,1 %igen Lösung des Polymerkonjugats aus Beispiel 7 dargestellt. Die Trübung wurde photometrisch bestimmt. Eine Trübung von 100 % entspricht einer vollständigen Ausfällung des Polymerkonjugats.

Zur Ausfüllung des erfindungsgemäßen Polymerkonjugats können der Lösung 2 - 90 Gew.-%, bevorzugt 5 - 70 Gew.-% - bezogen auf die Masse des gelösten Polymerkonjugats - Salz, bevorzugt als Feststoff aber auch in gelöster Form, zugegeben werden.

Als Lösungsmittel für die erfindungsgemäßen Polymerkonjugate eignen sich außer Wasser auch wäßrige Lösungen, die bis zu 20 Gew.-% eines mit Wasser vollständig und unbegrenzt mischbaren organischen Lösungsmittels wie Methanol, Ethanol oder Aceton enthalten.

Der Vorteil der erfindungsgemäßen Polymerkonjugate liegt hauptsächlich in ihrer Wasserlöslichkeit und ihrer einstellbaren reversiblen Ausfällbarkeit. Selbst nach mehrmaligem "Gebrauch", also nach mehrmaligem Auflösen und Wiederausfällen, nimmt die Aktivität der an diese Trägerpolymere gebundenen katalytisch aktiven Substanzen nur unwesentlich ab (s. Beispiel 9).

Mit Hilfe der erfindungsgemäßen Polymerkonjugate sind katalytische Reaktionen von immobilisierten

Enzymen
Coenzymen
Antikörpern
Antigenen
Affinitätsmaterialien
Aminosäuren
Metallen oder Metallkomplexen (Chiralika)

in homogener Phase möglich, wobei das eingesetzt katalytisch aktive Material wiedergewonnen und mehrmals eingesetzt werden kann. Dadurch werden die Nachteile der heterogenen Katalyse wie:

- Schlechte Ausnutzung des vorhandenen Katalysators
- Diffusionshemmung (gerade hochmolekulare Teilchen erreichen den Katalysator nur schlecht)
- Eventuelle Desaktivierung des Katalysators durch pH-Gradient

- Limitierung der Edukt- und Produktgröße durch die Porengröße des Trägermaterials
- Verminderung der Aktivität durch Porenverstopfung vermieden, und die Vorteile der homogenen Katalyse wie:
- Hohe Reaktionsgeschwindigkeit
- Abbau und Modifizierung hochmolekularer Substrate
- Herstellung hochmolekularer Produkte
- Reaktionen mit unlöslichen Produkten

können voll genutzt werden, ohne dabei auf die bisher übliche aufwendige Abtrennung zurückgreifen zu müssen.

Im folgenden wird die Erfindung anhand von Beispielen näher erläutert.

Beispiel 1

4,2 g N-Isopropylacrylamid, (NIPAM) und 0,76 g Acrylsäuremethylester (AME) werden in 50 ml entgastem Wasser gelöst. Die Monomerlösung wird auf 15 °C unter Sauerstoffausschluß temperiert. Um die Polymerisation zu starten, wird zunächst 1 ml 0,5 Gew.-% Ammoniumperoxodisulfat und 1 ml 0,5 Gew.-% Natriumthiosulfit als wäßrige Lösung zu der Monomerlösung gegeben.

Nach 96 Minuten wird die Polymerisation durch Einblasen von Luft abgebrochen.

Allgemeine Aufarbeitung:

Ohne zu rühren, erwärmt man die Reaktionslösung, bis eine Trübung entsteht, und fügt unter heftigem Rühren kristallines Natriumchlorid hinzu. Das Polymer fällt aus, man dekantiert vom Feststoff ab und fällt das Polymer noch zweimal aus wäßriger Lösung wie beschrieben aus. Ausbeute 65 % (Polymer 1)

Beispiel 2-4

Entsprechend Beispiel 1 werden Copolymere mit unterschiedlichen Monomergehalten entsprechend nachfolgender Tabelle 1 synthetisiert.

Tabelle 1

| Polmyer | Konzentration [g] | | Polymerisierungstemperatur °C | Reaktionszeit (min) | Ausbeute % | Zusammensetzung des Polymeren* Gew.-% |
|---|---|---|---|---|---|---|
| | NIPAM | AME | | | | |
| 1 | 4,2 | 0.76 | 15 | 96 | 65,1 | 92/8,1 |
| 2 | 3,6 | 1,25 | 5 | 65 | 8,9 | 73/27 |
| 3 | 3,25 | 1,32 | 15 | 253 | 85,9 | 83/17 |
| 4 | 3,22 | 1,66 | 15-5 | 240 | 52,1 | 69/31 |

* Die Polymerzusammensetzung wurde mittels Protonenresonanz-Spektroskopie in Deuterochloroform als Lösungsmittel bestimmt.

Beispiel 5

Man verfährt analog Beispiel 1 unter Verwendung der Monomere N-Isopropylacrylamid (4 g) und Methacrylamid (0,6 g). Nach 300 Minuten wird die Polymerisation durch Einblasen von Sauerstoff gestoppt. Ausbeute nach Reinigung 52,4 % (Polymer 5)

Beispiel 6-8

Herstellung von Copolymeren mit Oxirangruppen.

Man verfährt analog Beispiel 1 unter den in nachfolgender Tabelle 2 aufgeführten Reaktionsbedingungen:

Tabelle 2

| Polymer | NIPAM [g] | GMA [g] | Polymerisierungstemp. [°C] | Reaktionszeit [min] | Epoxidgehalt Gew.-% |
|---|---|---|---|---|---|
| 6 | 5,4 | 0,14 | 25 | 40 | 4 |
| 7 | 5,4 | 0,11 | 20 | 240 | 4 |
| 8 | 5,4 | 0,29 | 4 | 40 | 22 |

Die Polymerisation wurde in jeweils 60 ml Wasser mit 1 ml Ammoniumperoxodisulfat (0,5 Gew.-%), 1 ml Natriumthiosulfit (0,5 Gew.-%) für Polymer 6 und Polymer 7 bzw. 1 ml Ammoniumperoxodisulfat und 0,5 ml Natriumthiosulfit (für Polymer 8) durchgeführt.

Reinigung der Copolymere 6 - 8:

2 Teile der Polymerisationslösung werden mit 1 Teil Aceton versetzt. Unter starkem Rühren gibt man 12 Teile Diäthylether hinzu. Das Polymer fällt aus und die in der Etherphase gelösten Monomere werden abdekantiert. Die feuchte Polymermasse wird in wenig Aceton gelöst und in Diäthylether ausgetropft. Dieser Vorgang wird mehrmals wiederholt, das Produkt anschließend abgesaugt und im Vakuum bei Raumtemperatur getrocknet.

Bestimmung des Epoxidgehaltes:

Der Epoxidgehalt der erhaltenen Copolymere wurde titrimetrisch bestimmt. 0,5 g Copolymer werden in 50 ml destilliertem Wasser gelöst und mit 0,01 normaler Natronlauge auf pH 7,0 eingestellt. Anschließend gibt man 15 ml einer 0,13 molaren Thiosulfatlösung hinzu und hält durch Zugabe von 0,01 normaler Salzsäure den pH-Wert auf 7 konstant (Autotitration).

Beispiel 9

Jeweils 1 g Polymer aus den Beispielen 6 bis 8 werden in 100 ml 0,05 molaren Phosphatpuffers (pH 7,0) bei Raumtemperatur gelöst. Dazu gibt man 0,4 g (Beispiel 6), 0,47 g (Beispiel 7) und 0,5 g (Beispiel 8) Trypsin. Man rührt anschließend 24 Stunden bei 15 °C. Die Reinigung des Polymer-Enzym-Konjugats wird wie in Beispiel 1 beschrieben, durchgeführt. Die erhaltenen Aktivitäten, gemessen in Units pro Gramm Polymer, in Abhängigkeit von den Fällungen sind in Figur 3 gezeigt.

Die Enzymaktivität des Konjugats im Vergleich zu dem freien Trypsin wird an der hydrolytischen Spaltung von N-Benzoyl-L-Argininethylester in 0,05 molarem Tris-Puffer (pH 7,5/unter Zusatz von 5 mmol Calciumchlorid) bestimmt. Eine Unit ist definiert als: $10^{-6}$ Mol/min. hydrolysiertem N-Benzoyl-L-Argininethylesterhydrochlorid.

Beispiel 10

1 g Polymer 8 aus Beispiel 8 werden in 100 ml 2-[4-(2-Hydroxyethyl)-1-piperazinyl]-ethansulfonsäure-Puffer (HEPES-Puffer) bei Raumtemperatur gelöst (pH 7,3). Dazu gibt man 0,8 g Thermolysin und rührt 48 Stunden bei 4 °C. Anschließend wird die Lösung auf 30° erwärmt und durch Zugabe von Natriumchlorid das Polymer-Enzym-Konjugat ausgefällt. Dieser Vorgang wird so oft wiederholt (ca. 5 Mal) bis die UV-Absorption der Waschlösung bei 280 nm konstant bleibt.
Ausbeute: 1,15 g; enthält 30 Gew.-% Thermolysin
Zur Bestimmung der Aktivität wurde die hydrolytische Spaltung von 3-(2-Furylacryloyl)-glycyl-L-leucinamid (FAGLA) benutzt.

Dieses Verfahren wird in Biochemical and Biophysical Research Communications, Vol. 32, (1968), S. 326-332, J. Feder beschrieben.

Durchführung:

1,5 ml Substratlösung (2,45 $10^{-3}$ molar) in 0,001 molarem Phosphatpuffer (pH 7,2) werden 3-10 mg Polymer zugegeben. Nach 10 Minuten rühren bei Raumtemperatur gibt man 50 ml 1N Salzsäure zu. Die Spaltaktivität wird durch HPLC an einer Reverse Phase Säule bestimmt.

Beispiel 11

0,5 g Polymer aus Beispiel 8 werden in 50 ml 0,05 molarem Phosphatpuffer (pH 7,0) gelöst. Dazu gibt man 0,4 g Lipase aus Candida cylindracea und fixiert bei 5 °C. Das Polymer-Enzym-Konjugat wird entsprechend Beispiel 1 aufgearbeitet.

Beispiel 12

0.208 g Polymer aus Beispiel 8 werden in 40 ml Wasser, daß 5mM Calciumchlorid enthält, gelöst. Dazu gibt man ca 16 mg Cyclodextrin-glycosyltransferase (EC 2.3.1.19) aus Bacillus-macerans und inkubiert 18 Stunden bei 15 °C und 50 Stunden bei 5 °C.

Um das Polymer-Enzym-Konjugat von nicht gebundenem Enzym zu befreien, wird es 7 Mal entsprechend Beispiel 1 ausgefällt.

Die Aktivität der Cyclodextrin-glycosyltransferase wurde wie folgt bestimmt.

0.1 g Polymer-Enzym-Konjugat werden in 5 mM Phosphatpuffer pH 7.0 und 5 %iger Stärkelösung (Kartoffelstärke [(R)]Paselli) bei 25 °C gespalten.

Die Bestimmung der gebildeten α-, β- und γ-Cyclodextrine wurde mittels HPLC mit Acetonitril-Wasser (68:32) durchgeführt. Das Immobilisat zeigt die gleiche Produktverteilung von α-, β- und γ-Cyclodextrin wie das lösliche Enzym. Für das Enzym Polymer-Konjugat wurde so eine Aktivität von 450 units/g Polymer erhalten.

**Patentansprüche**

**1.** Wasserlösliches Polymerkonjugat, bestehend aus einem Trägerpolymeren und einer chemisch daran gebundenen katalytisch aktiven Verbindung, dadurch gekennzeichnet, daß das Polymerkonjugat aus einem wasserlöslichen, aus wäßriger Lösung durch Temperaturerhöhung und/oder Salzzugabe reversibel ausfällbaren Trägerpolymer, welches aus mindestens einer wiederkehrenden Monomereinheit ausgewählt aus den Verbindungen der Formel I

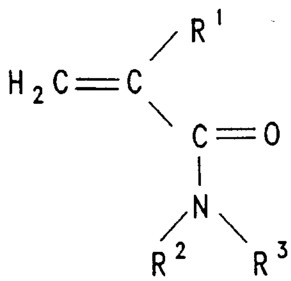

(I)

wobei

$R^1$ und $R^3$ gleich oder verschieden sind und Wasserstoff oder geradkettiges oder verzweigtes $C_1$-$C_3$-Alkyl und

$R^2$ geradkettiges oder verzweigtes $C_1$-$C_3$-Alkyl bedeutet

und mindestens einer weiteren wiederkehrenden Monomereinheit, ausgewählt aus den Verbindungen der Formel II, III und IV,

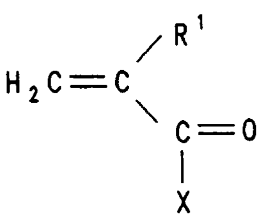

(II)

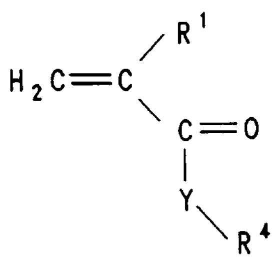

(III)

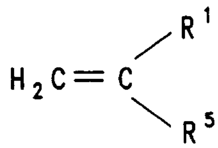

(IV)

wobei

$R^1$ wie zu Formel I angegeben definiert ist,

$R^4$ eine hydrophobe Gruppe ist und geradkettiges $C_1$-$C_6$-Alkyl, welches im Falle der $C_3$-$C_6$-Alkylreste auch verzweigt oder cyclisch sein kann,

$R^5$ eine aktivierte Gruppe ist und 3,4-Epoxicyclohexyl oder ein Substituent der Formel V,

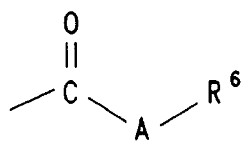

(V)

wobei

A Sauerstoff, Schwefel oder eine NH-Gruppe und

$R^6$ ein $-(CH_2)_n$-Z-Rest,

worin

n eine Zahl von 1 bis 6 und

Z Amin, Hydroxyl, Carboxyl, Carbonyl oder 1,2-Epoxyethan bedeutet,

ist,

X eine hydrophile Gruppe ist und eine OH-, $NH_2$- oder SH-Gruppe und

Y Sauerstoff oder Schwefel

bedeutet,

besteht und aus einer katalytisch aktiven Verbindung besteht.

**2.** Polymerkonjugat nach Anspruch 1, dadurch gekennzeichnet, daß das Trägerpolymer einen Fällpunkt von 10-80 °C besitzt.

**3.** Polymerkonjugat nach Anspruch 1, dadurch gekennzeichnet, daß die katalytisch aktive Verbindung ein Enzym, Coenzym, Antigen, Antikörper, Metall, Metallkomplex, Affinitätsmaterial, oder eine Aminosäure ist.

**4.** Polymerkonjugat nach Anspruch 1, dadurch gekennzeichnet, daß das Trägerpolymere aus einer wiederkehrenden Monomereinheit, gemäß Formel I sowie aus einer weiteren wiederkehrenden Monomer-Einheit gemäß Formel IV und 0 bis 2 weiteren wiederkehrenden Monomereinheiten, ausgewählt aus den Verbindungen der Formel II und III besteht.

**5.** Polymerkonjugat nach Anspruch 5, dadurch gekennzeichnet daß das Trägerpolymere eine wiederkehrende Monomereinheit, ausgewählt aus den Verbindungen der Formel II und III enthält.

**6.** Polymerkonjugat nach Anspruch 1, dadurch gekennzeichnet, daß

$R^1$ Wasserstoff, Methyl oder Ethyl,

$R^2$ n- oder i-Propyl,

$R^3$ Wasserstoff, Methyl oder Ethyl,

$R^4$ geradkettiges oder verzweigtes $C_1$-$C_4$-Alkyl,

$R^5$ 3,4-Epoxycyclohexan oder ein Substituent der Formel VI,

wobei

$R^6$ ein- $(CH_2)_n$-Z-Rest, mit n = 1 bis 3 und

Z Carbonyl oder 1,2-Epoxyethan bedeutet und

A Sauerstoff oder eine NH-Gruppe ist,

X eine OH- oder $NH_2$-Gruppe und

Y Sauerstoff

bedeutet.

**7.** Polymerkonjugat nach Anspruch 1, dadurch gekennzeichnet, daß die Verbindung gemäß Formel I N-Isopropylacrylamid oder N-Isopropylmethacrylamid, die gemäß Formel II Methacrylamid, die gemäß Formel III Acrylsäuremethylester und die gemäß Formel IV Glycidylmethacrylat oder Glycidylacrylat ist.

**8.** Polymerkonjugat nach Anspruch 1, dadurch gekennzeichnet, daß der Gewichtsanteil mindestens einer der Verbindungen gemäß Formel II, III und IV 0,2-80 %, bezogen auf das Gewicht der Monomereinheit gemäß Formel I beträgt.

**9.** Polymerkonjugat nach Anspruch 6, dadurch gekennzeichnet, daß der Gewichtsanteil der Monomereinheit gemäß Formel IV 0,2-15 %, bezogen auf das Gewicht der Monomereinheiten gemäß Formel I und II oder III, beträgt.

**10.** Polymerkonjugat gemäß Anspruch 5, dadurch gekennzeichnet, daß die katalytisch wirksame Verbindung über die aktivierte Gruppe der Monomereinheit gemäß Formel IV chemisch gebunden ist.

**11.** Verfahren zur Herstellung der Polymerkonjugate gemäß Anspruch 1, dadurch gekennzeichnet, daß man das wasserlösliche, aus wäßriger Lösung durch Temperaturerhöhung und/oder Salzzugabe reversibel ausfällbare Trägerpolymer mit einer katalytisch wirksamen Verbindung umsetzt.

**12.** Verwendung der Polymerkonjugate gemäß Anspruch 1 in der homogenen Katalyse.

**13.** Verwendung der Polymerkonjugate gemäß Anspruch 1 in der homogenen Biokatalyse.

## Claims

**1.** A water-soluble polymer conjugate, composed of a carrier polymer and of a catalytically active compound chemically bonded thereto, wherein the polymer conjugate is composed of a water-soluble carrier polymer which is reversibly precipitable from aqueous solution by raising the temperature and/or addition of salt, and which is composed of at least one repeating monomer unit selected from the compounds of the formula I

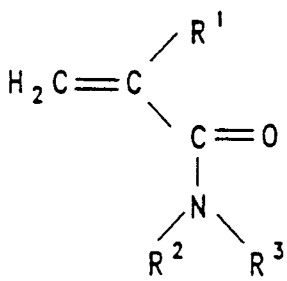

(I)

where
$R^1$ and $R^3$ are identical or different and denote hydrogen or straight-chain or branched $C_1$-$C_3$-alkyl, and
$R^2$ denotes straight-chain or branched $C_1$-$C_3$-alkyl,
and of at least one other repeating monomer unit selected from the compounds of the formula II, III and IV

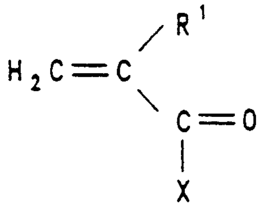

(II)

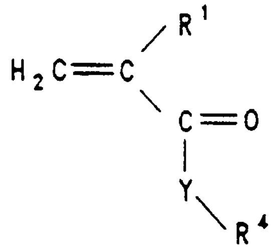

(III)

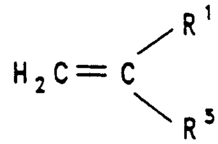

(IV)

where
$R^1$ is defined as indicated for formula I,

$R^4$ is a hydrophobic group and denotes straight-chain $C_1$-$C_6$-alkyl which, in the case of $C_3$-$C_6$-alkyl radicals, can also be branched or cyclic,
$R_5$ is an activated group and is 3,4-epoxycyclohexyl or a substituent of the formula V

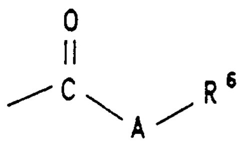

(V)

where
A is oxygen, sulfur or an NH group, and
$R^6$ is a $-(CH_2)_n-Z$ radical,
in which
n denotes a number from 1 to 6, and
Z denotes amino, hydroxyl, carboxyl, carbonyl or 1,2-epoxyethane,
X is a hydrophilic group and denotes an OH, $NH_2$ or SH group, and
Y denotes oxygen or sulfur,
and of a catalytically active compound.

**2.** A polymer conjugate as claimed in claim 1, wherein the carrier polymer has a precipitation point of 10-80 ° C.

**3.** A polymer conjugate as claimed in claim 1, wherein the catalytically active compound is an enzyme, coenzyme, antigen, antibody, metal, metal complex, affinity material or an amino acid.

**4.** A polymer conjugate as claimed in claim 1, wherein the carrier polymer is composed of a repeating monomer unit of the formula I and of another repeating monomer unit of the formula IV and of 0 to 2 further repeating monomer units selected from the compounds of the formula II and III.

**5.** A polymer conjugate as claimed in claim 4, wherein the carrier polymer contains a repeating monomer unit selected from the compounds of the formula II and III.

**6.** A polymer conjugate as claimed in claim 1, wherein
$R^1$ denotes hydrogen, methyl or ethyl,
$R^2$ denotes n- or i-propyl,
$R^3$ denotes hydrogen, methyl or ethyl,
$R^4$ denotes straight-chain or branched $C_1$-$C_4$-alkyl,
$R_5$ denotes 3,4-epoxycyclohexane or a substituent of the formula VI,
where
$R_6$ denotes a $-(CH_2)_n-Z$ radical with n = 1 to 3,
and
Z denotes carbonyl or 1,2-epoxyethane, and
A is oxygen or an NH group,
X denotes an OH or $NH_2$ group, and
Y denotes oxygen.

**7.** A polymer conjugate as claimed in claim 1, wherein the compound of the formula I is N-isopropylacrylamide or N-isopropylmethacrylamide, that of the formula II is methacrylamide, that of the formula III is methyl acrylate, and that of the formula IV is glycidyl methacrylate or glycidyl acrylate.

**8.** A polymer conjugate as claimed in claim 1, wherein the proportion by weight of at least one of the compounds of the formula II, III and IV is 0.2-80% based on the weight of the monomer unit of the formula I.

**9.** A polymer conjugate as claimed in claim 6, wherein the proportion by weight of the monomer unit of the formula IV is 0.2-15% based on the weight of the monomer units of the formula I and II or III.

**10.** A polymer conjugate as claimed in claim 5, wherein the catalytically active compound is chemically bonded via the activated group of the monomer unit of the formula IV.

**11.** A process for the preparation of the polymer conjugates as claimed in claim 1, which comprises reacting the water-soluble carrier polymer, which is reversibly precipitable from aqueous solution by raising the temperature and/or addition of salt, with a catalytically active compound.

**12.** The use of the polymer conjugates as claimed in claim 1 in homogeneous catalysis.

**13.** The use of the polymer conjugates as claimed in claim 1 in homogeneous biocatalysis.

## Revendications

**1.** Conjugué de polymère soluble dans l'eau, constitué d'un polymère de support et d'un composé catalytiquement actif lié chimiquement à celui-ci, caractérisé en ce que le conjugué de polymère consiste en un polymère de support soluble dans l'eau, précipitable de façon réversible, à partir d'une solution aqueuse, par élévation de la température et/ou par une addition de sel, qui est constitué d'au moins un motif monomère répétitif de formule I

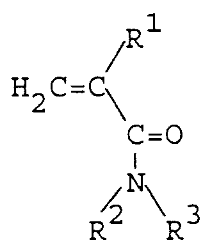

(I)

dans laquelle

- $R^1$ et $R^3$ sont identiques ou différents et représentent un atome d'hydrogène ou un groupe alkyle ne $C_1$-$C_3$ à chaîne droite ou ramifiée, et
- $R^2$ représente un groupe alkyle en $C_1$-$C_3$ à chaîne droite ou ramifiée,

et d'au moins un autre motif monomère répétitif choisi parmi les composés de formules II, III et IV

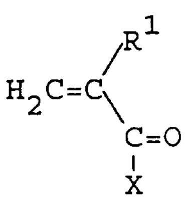

(II)

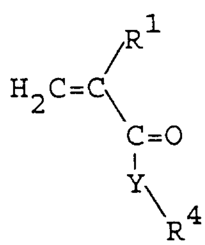

(III)

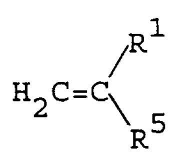

(IV)

formules dans lesquelles

- $R^1$ est défini comme indiqué à propos de la formule I,
- $R^4$ est un groupe hydrophobe et représente un groupe alkyle en $C_1$-$C_6$ à chaîne droite qui, dans le cas des groupes alkyle en $C_3$-$C_6$, peut également être ramifié ou cyclique,
- $R^5$ est un groupe activé et représente le groupe 3,4-époxycyclohexyle ou un substituant de formule V

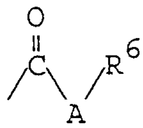

(V)

dans laquelle

A représente un atome d'oxygène, de soufre ou un groupe NH, et

$R^6$ est un radical $-(CH_2)_n-Z$ dans lequel

n représente un nombre allant de 1 à 6 et

Z représente un groupe amino, hydroxy, carboxy, carbonyle ou 1,2-époxyéthane,

X est un groupe hydrophile et représente un groupe OH, $NH_2$ ou SH, et

Y représente un atome d'oxygène ou de soufre,

et d'un composé catalytiquement actif.

2. Conjugué de polymère selon la revendication 1, caractérisé en ce que le polymère de support a un point de précipitation de 10-80 °C.

3. Conjugué de polymère selon la revendication 1, caractérisé en ce que le composé catalytiquement actif est une enzyme, une coenzyme, un antigène, un anticorps, un métal, un complexe métallique, un matériau d'affinité ou un aminoacide.

4. Conjugué de polymère selon la revendication 1, caractérisé en ce que le polymère de support est constitué d'un motif monomère répétitif de formule I ainsi que d'un autre motif monomère répétitif de formule IV, et de 0 à 2 autres motifs monomères répétitifs, choisis parmi les composés de formules II et III.

5. Conjugué de polymère selon la revendication 4, caractérisé en ce que le polymère de support contient un motif monomère répétitif choisi parmi les composés de formules II et III.

6. Conjugué de polymère selon la revendication 1, caractérisé en ce que

$R^1$ représente un atome d'hydrogène ou le groupe méthyle ou éthyle,

$R^2$ représente le groupe n- ou isopropyle,

$R^3$ représente un atome d'hydrogène ou le groupe méthyle ou éthyle,

$R^4$ représente un groupe alkyle en $C_1$-$C_4$ à chaîne droite ou ramifiée,

$R^5$ représente le groupe 3,4-époxycyclohexane ou un substituant de formule V dans lequel

$R^6$ est un radical $-(CH_2)_n-Z$, avec n = 1 à 3 et

Z représentant le groupe carbonyle ou 1,2-époxyéthane, et

A représente un atome d'oxygène ou le groupe NH,

X représente le groupe OH ou $NH_2$ et

Y représente un atome d'oxygène.

7. Conjugué de polymère selon la revendication 1, caractérisé en ce que le composé de formule I est le N-isopropylacrylamide ou le N-isopropylméthacrylamide, le composé de formule II est le méthacrylamide, le composé de formule III est l'acrylate de méthyle et le composé de formule IV est le méthacrylate de glycidyle ou l'acrylate de glycidyle.

8. Conjugué de polymère selon la revendication 1, caractérisé en ce que la proportion pondérale d'au moins l'un des composés de formules II, III et IV va de 0,2 à 80 %, par rapport au poids du motif monomère de formule I.

9. Conjugué de polymère selon la revendication 6, caractérisé en ce que la proportion pondérale du motif monomère de formule IV va de 0,2 à 15 %, par rapport au poids des motifs monomères de formules I et II ou III.

10. Conjugué de polymère selon la revendication 5, caractérisé en ce que le composé catalytiquement actif est lié chimiquement par l'intermédiaire du groupe activé du motif monomère de formule IV.

11. Procédé pour la préparation des conjugués de polymère selon la revendication 1, caractérisé en ce que l'on fait réagir avec un composé catalytiquement actif le polymère de support soluble dans l'eau, précipitable de façon réversible à partir d'une solution aqueuse, par élévation de la température et/ou addition d'un sel.

12. Utilisation des conjugués de polymères selon la revendication 1, en catalyse homogène.

13. Utilisation des conjugués de polymères selon la revendication 1, en biocatalyse homogène.

Fig. 1

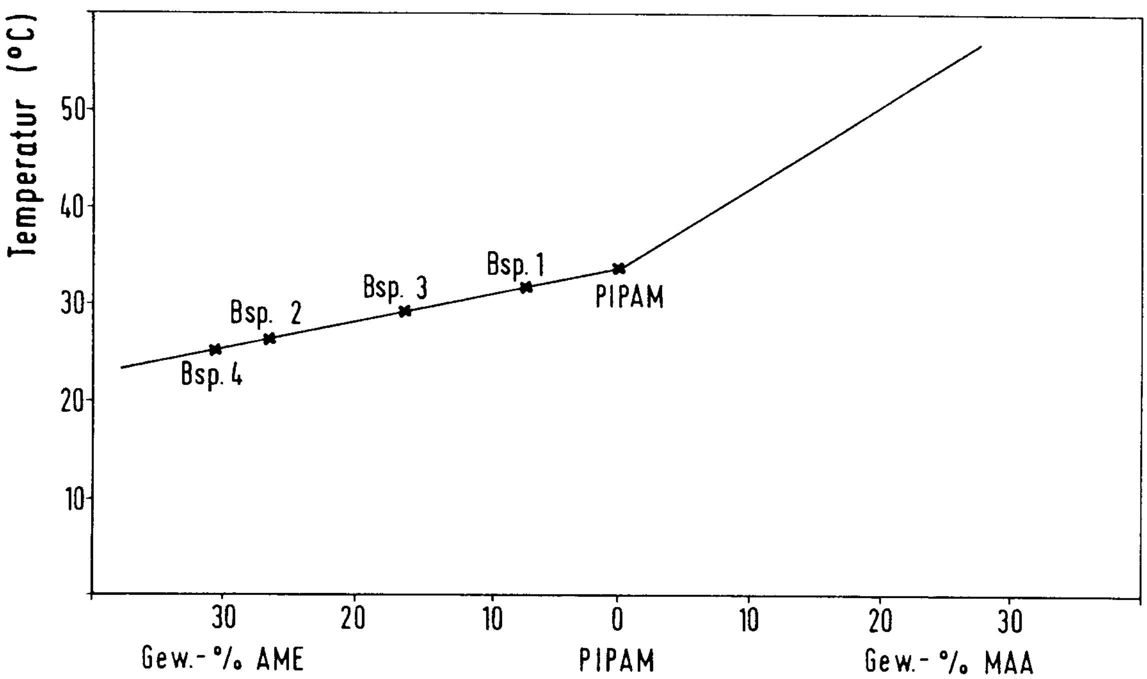
Temperatur (°C)
50
40
30
20
10
Bsp. 1
Bsp. 3
Bsp. 2
Bsp. 4
PIPAM
30
20
10
0
10
20
30
Gew.-% AME
PIPAM
Gew.-% MAA

Fig. 2

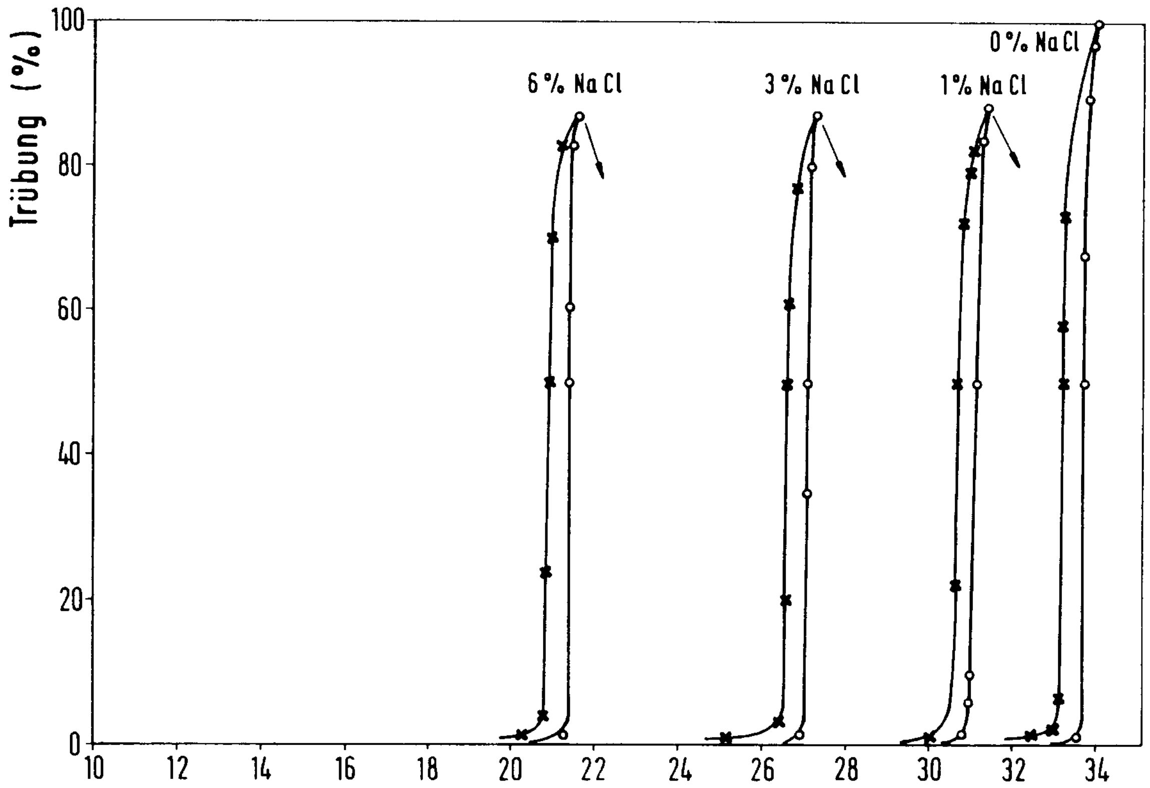
Trübung (%)
100
80
60
40
20
0
6 % NaCl
3 % NaCl
1% NaCl
0 % NaCl
10
12
14
16
18
20
22
24
26
28
30
32
34
Temperatur (°C)

Fig. 3

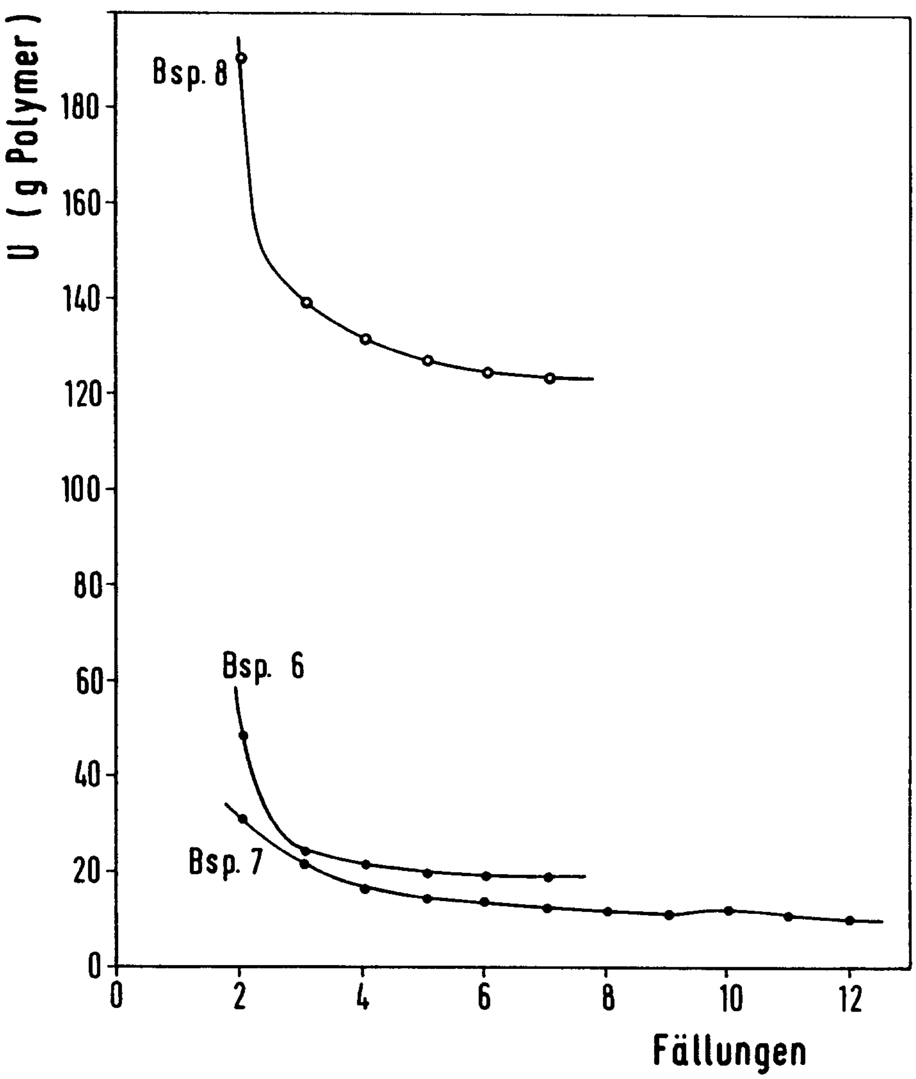
U (g Polymer)
180
160
140
120
100
80
60
40
20
0
Bsp. 8
Bsp. 6
Bsp. 7
0
2
4
6
8
10
12
Fällungen